# EUROPEAN PATENT APPLICATION

(11) **EP 4 745 987 A1**
(43) Date of publication of application: **20.05.2026**
(21) Application number: 24212731.4
(22) Date of filing: 13.11.2024
(51) Int. Cl.: G16H 50/20, G16H 50/30, G16H 50/70

(54) **TRAINING METHOD AND APPARATUS FOR PREDICTIVE AND INTERPRETIVE MODEL, AND COMPUTER DEVICE**

(71) Applicant: Haleon US Holdings LLC, Wilmington, DE 19808 (US)
(72) Inventor: SONG, Yuedong, Pudong New Area, Shanghai (CN); CHEN, Yao, Wuzhong District Suzhou, Jiangsu (CN); ZHANG, Wei, Pudong New Area, Shanghai (CN); BEAUDRY, Scott Alexander, Pudong New Area, Shanghai (CN); ZHANG, Cheng, Pudong New Area, Shanghai (CN)
(74) Representative: Haleon Patent Department

(57) **Abstract**

The present disclosure relates to a training method and apparatus for a predictive and interpretive model, and a computer device. The method includes: acquiring sample data of a plurality of users; ranking feature importance of a plurality of detailed action index parameters indicating the plurality of detailed actions based on a feature screening algorithm, and screening out a plurality of important index parameters according to ranking result; inputting the plurality of important index parameters into a trained prediction sub-model to output a prediction result indicating a health degree of joints, training by using a plurality of important index parameters obtained from sample data of part of users, and testing by using a plurality of important index parameters obtained from sample data of remaining users to obtain the trained prediction sub-model; inputting the prediction result into an interpretation sub-model to perform interpretation on the prediction result, to determine a contribution degree of the plurality of important index parameters to the prediction result; and obtaining a trained predictive and interpretive model based on the trained prediction sub-model and the interpretation sub-model.

## Description

### TECHNICAL FIELD

The present disclosure relates to the field of machine learning prediction, and more particularly, to a training method and apparatus for a predictive and interpretive model for evaluating a health degree of joints, and a computer device.

### BACKGROUND

Conventionally, to evaluate a health degree of joint organs such as knee and hip joints, the evaluated person needs to complete a WOMAC rating scale, if a total score of the scale is high, it means that the overall sub-healthy likelihood of knee and hip joints is high. Then the evaluated person needs to further do several sets of knee and hip joints related actions to evaluate whether some finer indicators are within healthy ranges.

However, according to performance of experimental samples, the researchers have found that the value ranges of sub-healthy and healthy people in knee and hip joints are highly overlapping in respective detailed action indexes, so it is difficult to distinguish them by defining thresholds.

As one of the important branches in the field of artificial intelligence, machine learning is widely applied in data mining, image recognition, natural language processing and other fields. However, the current machine learning algorithms cannot effectively evaluate the health degree of j oint organs.

### SUMMARY

In order to better evaluate the health degree of joints, the present disclosure provides a training method of a predictive and interpretive model for evaluating a health degree of joints, the health degree of joints is evaluated by an automatic algorithm through the trained predictive and interpretive model, and the result predicted by the model is automatically interpreted.

According to an aspect of the present disclosure, there is provided a training method of a predictive and interpretive model for evaluating a health degree of joints, the method including: acquiring sample data of a plurality of users, wherein sample data of each user includes a plurality of action video data of a plurality of sets of actions performed by each user on joints, and each action of the plurality of sets of actions includes a plurality of detailed actions; ranking feature importance of a plurality of detailed action index parameters indicating the plurality of detailed actions based on a feature screening algorithm, and screening out a certain number of detailed action index parameters from the plurality of detailed action index parameters according to a ranking result as a plurality of important index parameters; inputting the plurality of important index parameters into a trained prediction sub-model to output a probability-based prediction result indicating a health degree of joints of each user, wherein a plurality of important index parameters obtained from sample data of part of users among the plurality of users are used as training data to train a prediction sub-model, and a plurality of important index parameters obtained from sample data of remaining users among the plurality of users are used as verification data to test the prediction sub-model to obtain the trained prediction sub-model; inputting the prediction result into an interpretation sub-model to perform a feature contribution degree interpretation on the prediction result, to determine a contribution degree of the plurality of important index parameters to the prediction result; and obtaining a trained predictive and interpretive model based on the trained prediction sub-model and the interpretation sub-model.

According to an embodiment of the present disclosure, the method may further include: performing action detection on each of the plurality of action video data through a key point detection algorithm to obtain the quantized plurality of detailed action index parameters indicating different detailed actions in the plurality of sets of actions.

According to an embodiment of the present disclosure, the method may further include: performing abnormal value determination on the plurality of detailed action index parameters, and removing the detailed action index parameters determined as abnormal values.

According to an embodiment of the present disclosure, abnormal value determination may be performed on the plurality of detailed action index parameters based on an IQR statistical measurement method, and the IQR statistical measurement method includes: calculating a first value by subtracting 1.5 IQR from the 25th percentile value and a second value by adding 1.5 IQR to the 75th percentile value, and determining a value smaller than the first value and a value larger than the second value as the abnormal values, wherein IQR refers to a value obtained by subtracting the 25th percentile value from the 75th percentile value.

According to an embodiment of the present disclosure, the feature screening algorithm may include a Boruta algorithm, and screening out the plurality of important index parameters includes: constructing a plurality of shadow features corresponding to the plurality of detailed action index parameters; ranking feature importance of the plurality of detailed action index parameters with feature importance of the plurality of shadow features as a reference basis; and after multiple iterations, screening out the certain number of detailed action index parameters from the plurality of detailed action index parameters according to the ranking result as the plurality of important index parameters.

According to an embodiment of the present disclosure, the prediction sub-model may include a LightGBM prediction model, and training of the LightGBM prediction model includes: determining an optimal superparameter combination of the LightGBM prediction model by constructing a plurality of value intervals for a plurality of sets of superparameters of the LightGBM prediction model to run a grid search strategy and by performing k-fold cross-validation on the LightGBM prediction model.

According to an embodiment of the present disclosure, the k-fold cross-validation may include 5-fold cross-validation and 10-fold cross-validation.

According to an embodiment of the present disclosure, performing a feature contribution degree interpretation on the prediction result may include: calculating a feature contribution degree of each of the plurality of important index parameters based on a SHAP algorithm; and interpreting a contribution degree of each important index parameter to the prediction result according to the feature contribution degree of each important index parameter.

According to an embodiment of the present disclosure, performing a feature contribution degree interpretation on the prediction result may further include: when the value of the feature contribution degree of an important index parameter is greater than 0, it indicates that the important index parameter has a positive influence on the prediction result; and when the value of the feature contribution degree of the important index parameter is less than 0, it indicates that the important index parameter has a reverse influence on the prediction result, wherein the greater the absolute value of the value of the feature contribution degree of the important index parameter, the greater the influence of the important index parameter on the prediction result.

According to an embodiment of the present disclosure, the joints may include at least one or more of shoulder joint, elbow joint, hip joint, wrist joint, knee joint and ankle joint.

According to an embodiment of the present disclosure, the plurality of sets of actions may at least include: crouching, knee joint flexion in the left and right directions, hip joint flexion and extension in the left and right directions, knee lifting and alternately patting the knees with left and right hands, and standing on one foot.

According to an embodiment of the present disclosure, the plurality of detailed action index parameters may at least include: an average time duration of standing-crouching-standing, an average minimum angle that the knee joint can reach at the time of crouching, a minimum angle that the knee joint can reach at the time of a right knee bending action, a minimum angle that the knee joint can reach at the time of a left knee bending action, a maximum angle that the hip joint can reach at the time of a right hip flexion action, a maximum angle that the hip joint can reach at the time of a left hip flexion action, a maximum angle that the hip joint can reach at the time of a right hip extension action, a maximum angle that the hip joint can reach at the time of a left hip extension action, a maximum angle which the right leg can reach relative to the vertical direction when lifting the knees and alternately patting the knees with the left and right hands, a maximum angle which the left leg can reach relative to the vertical direction when lifting the knees and alternately patting the knees with the left and right hands, a maximum angle which the left leg can reach relative to the vertical direction when standing on one foot with the right foot, a maximum angle which the right leg can reach relative to the vertical direction when standing on one foot with the left foot, a maintenance time duration when standing on one foot with the right foot, and a maintenance time duration when standing on one foot with the left foot.

According to an embodiment of the present disclosure, the plurality of important index parameters may at least include: an average time duration of standing-crouching-standing, an average minimum angle that the knee joint can reach at the time of crouching, a minimum angle that the knee joint can reach at the time of a right knee bending action, a minimum angle that the knee joint can reach at the time of a left knee bending action, a maximum angle which the right leg can reach relative to the vertical direction when lifting the knees and alternately patting the knees with the left and right hands, a maximum angle which the left leg can reach relative to the vertical direction when lifting the knees and alternately patting the knees with the left and right hands, a maintenance time duration when standing on one foot with the right foot, and a maintenance time duration when standing on one foot with the left foot.

According to another aspect of the present disclosure, there is provided a computer-implemented method for evaluating a health degree of joints, the method may include: acquiring a plurality of action video data of a plurality of sets of actions performed by a user on joints; inputting the plurality of action video data into a predictive and interpretative model; predicting a health degree of joints of the user based on the predictive and interpretive model to output a probability-based prediction result indicating the health degree of joints of the user; and performing a feature contribution degree interpretation on the prediction result to output a feature contribution degree ranking of detailed action index parameters corresponding to each action among the plurality of sets of actions, wherein the predictive and interpretive model is obtained based on the method according to the above-described training method of a predictive and interpretive model for evaluating a health degree of joints.

According to an embodiment of the present disclosure, the method may further include: determining an influence of respective actions among the plurality of sets of actions on the health degree of joints of the user, based on the prediction result and the feature contribution ranking.

According to an embodiment of the present disclosure, the method may further include: providing customized sports or exercise suggestions for the user according to the prediction result and the feature contribution ranking.

According to another aspect of the present disclosure, there is provided a training apparatus for a predictive and interpretive model for evaluating a health degree of joints, the apparatus including: a memory in which computer-executable instructions are stored; and a processor coupled to the memory, the processor being configured to: acquire sample data of a plurality of users, wherein sample data of each user includes a plurality of action video data of a plurality of sets of actions performed by each user on joints, and each action of the plurality of sets of actions includes a plurality of detailed actions; rank feature importance of a plurality of detailed action index parameters indicating the plurality of detailed actions based on a feature screening algorithm, and screen out a certain number of detailed action index parameters from the plurality of detailed action index parameters according to a ranking result as a plurality of important index parameters; input the plurality of important index parameters into a trained prediction sub-model to output a probability-based prediction result indicating a health degree of joints of each user, wherein a plurality of important index parameters obtained from sample data of part of users among the plurality of users are used as training data to train a prediction sub-model, and a plurality of important index parameters obtained from sample data of remaining users among the plurality of users are used as verification data to test the prediction sub-model to obtain the trained prediction sub-model; input the prediction result into an interpretation sub-model to perform a feature contribution degree interpretation on the prediction result, to determine a contribution degree of the plurality of important index parameters to the prediction result; and obtain a trained predictive and interpretive model based on the trained prediction sub-model and the interpretation sub-model.

According to another aspect of the present disclosure, there is provided an apparatus for evaluating a health degree of joints, the apparatus including: an acquisition unit configured to acquire a plurality of action video data of a plurality of sets of actions performed by a user on joints; an input unit configured to input the plurality of action video data into a predictive and interpretative model, wherein the predictive and interpretative model is obtained based on the above-described training method of a predictive and interpretive model for evaluating a health degree of joints; a processing unit configured to predict a health degree of joints of the user based on the predictive and interpretive model to output a probability-based prediction result indicating the health degree of joints of the user, the processing unit is further configured to perform a feature contribution degree interpretation on the prediction result to output a feature contribution degree ranking of detailed action index parameters corresponding to each action among the plurality of sets of actions; and a display unit configured to display the prediction result and the feature contribution ranking.

According to another aspect of the present disclosure, there is provided a computer program product, wherein computer-readable instructions are stored on the computer program product, the computer-readable instructions can implement the above-described training method of a predictive and interpretive model for evaluating a health degree of joints and the above-described computer-implemented method for evaluating a health degree of joints when executed by a processor.

Therefore, the training method and apparatus for a predictive and interpretive model for evaluating a health degree of joints, and the computer device according to the embodiments of the present disclosure implement automatic evaluation of the health degree of joints by using the technology based on feature screening and interpretive machine learning, and solve the problem that detailed action index parameters of a healthy joint set and a sub-healthy joint set are highly overlapping that cannot be solved by the original threshold method.

### BRIEF DESCRIPTION OF THE DRAWINGS

In order to more clearly explain the technical solutions of the embodiments of the present disclosure, the drawings needed in the description of the embodiments will be briefly introduced below. Obviously, the drawings in the following description are only some exemplary embodiments of the present disclosure. For those of ordinary skill in the art, other drawings may be further obtained according to these drawings without any creative efforts.
FIG. 1 shows a flowchart of a training method of a predictive and interpretive model for evaluating a health degree of joints according to an embodiment of the present disclosure;
FIG. 2 shows a schematic diagram of a plurality of sets of video actions regarding knee and hip joints according to an embodiment of the present disclosure;
FIG. 3 shows a feature distribution diagram based on a Boruta algorithm according to an embodiment of the present disclosure;
FIG. 4 shows a schematic diagram of a LightGBM prediction model according to an embodiment of the present disclosure;
FIG. 5 shows a schematic diagram of a prediction result indicating a health degree of joints of a user and a feature contribution degree of respective important index parameters according to an embodiment of the present disclosure;
FIG. 6 shows a flowchart of a method for evaluating a health degree of joints according to an embodiment of the present disclosure;
FIG. 7 shows a block diagram of a training apparatus of a predictive and interpretive model for evaluating a health degree of joints according to an embodiment of the present disclosure; and
FIG. 8 shows a schematic diagram of an apparatus for evaluating a health degree of joints according to an embodiment of the present disclosure.

### DETAILED DESCRIPTION OF THE EMBODIMENTS

In order to make the objectives, technical solutions and advantages of the embodiments of the present disclosure clearer, the technical solutions of the embodiments of the present disclosure will be described in a clear and complete way below with reference to the drawings. Obviously, the described embodiments are only part of the embodiments of the present disclosure, rather than all the embodiments of the present disclosure, all the other embodiments obtained by those of ordinary skill in the art based on the described embodiments of the present disclosure without paying creative efforts fall into the protection scope of the present disclosure.

Unless otherwise defined, technical terms or scientific terms used in the present disclosure shall have common meaning known by those skilled in the art to which the present disclosure pertains. Words and expressions such as "first", "second" and the like used in the present disclosure do not denote any sequence, quantity or priority, but only intend to distinguish different components. Words such as "include", "contain" and the like refer to that an element or an object before this word contains all the elements or objects listed thereinafter or alternatives thereof, without excluding other elements or objects. Words such as "connected", "connecting" and the like are not restricted to physical or mechanical connections, but may include electrical connections, regardless of direct or indirect connections. Words such as "up", "below", "left", "right", etc., are only used to denote relative positional relationship, once an absolute position of the described object changes, the relative positional relationship may probably change correspondingly. In order to keep the following description of the embodiments of the present disclosure clear and concise, the detailed description of some known functions and known components is omitted in the present disclosure.

The flow chart is used in the present disclosure to illustrate steps of the method according to the embodiments of the present disclosure. It should be understood that the preceding or subsequent steps may not be necessarily performed precisely in order. Instead, the respective steps may be processed in a reverse order or simultaneously. At the same time, other operations may be added to these processes, or one or several steps may be removed from these processes.

In the specification and drawings of the present disclosure, elements are described in singular or plural forms according to the embodiments. However, the singular and plural forms are appropriately selected for the proposed situations only for convenience of explanation, not intended to limit the present disclosure. Therefore, singular forms may include plural forms, and plural forms may also include singular forms, unless the context clearly indicates otherwise.

Next, the training method and apparatus for a predictive and interpretive model for evaluating a health degree of joints, and the computer device provided by the present disclosure will be described in detail with reference to the drawings.

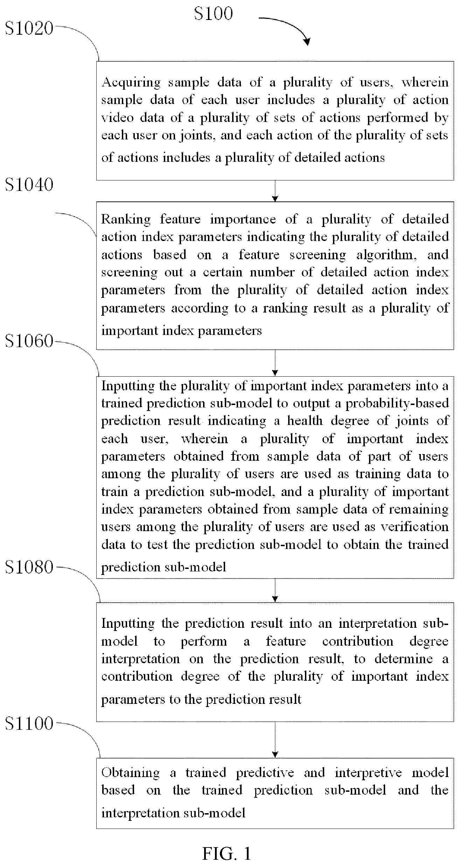

FIG. 1 shows a flowchart S100 of a training method of a predictive and interpretive model for evaluating a health degree of joints according to an embodiment of the present disclosure. Respective steps of the training method of a predictive and interpretive model for evaluating a health degree of joints according to the embodiment of the present disclosure will be described in detail below with reference to FIG. 1.

First, as shown in FIG. 1, the flowchart 5100 of a training method of a predictive and interpretive model for evaluating a health degree of joints according to the present disclosure may include respective steps shown according to processes S1020, S1040, S1060, S1080 and S1100.

In process S1020, sample data of a plurality of users may be acquired, wherein sample data of each user includes a plurality of action video data of a plurality of sets of actions performed by each user on joints, and each action of the plurality of sets of actions includes a plurality of detailed actions.

According to the embodiment of the present disclosure, the joints may include at least one or more of shoulder joint, elbow joint, hip joint, wrist joint, knee joint and ankle joint. In the embodiment of the present disclosure, description will be provided with knee and hip joints as an example.

For example, the user may perform a plurality of sets of actions for testing the health degree of knee and hip joints, such as crouching, sitting-standing, flexion and extension of hip joint, etc. Crouching is taken as an example, the user needs to complete a series of video actions when performing a crouching action, including three detailed actions of standing- crouching-standing. Sitting-standing is taken as an example, the user also needs to complete a series of video actions when performing a sitting-standing action, including three detailed actions of sitting-standing-sitting.

According to the embodiment of the present disclosure, action detection may be performed on each of the plurality of action video data through a key point detection algorithm to obtain the quantized plurality of detailed action index parameters indicating different detailed actions in the plurality of sets of actions.

The key point detection technology is one of the basic research directions in computer vision, and its main goal is to identify key points of objects in images or videos, such as joint positions and facial feature points and so on of human bodies. The key point detection technology plays an important role in many applications, such as human posture estimation, facial feature detection, hand action recognition and so on. In human posture estimation, the key points usually refer to the joint positions of human bodies, such as shoulders, elbows, knee joints and hip joints.

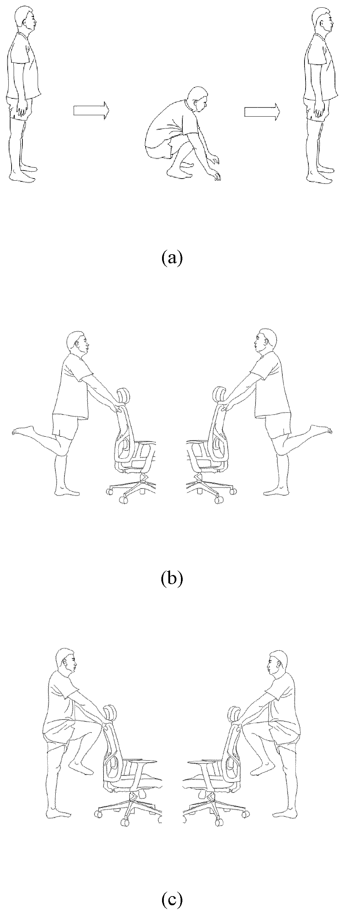

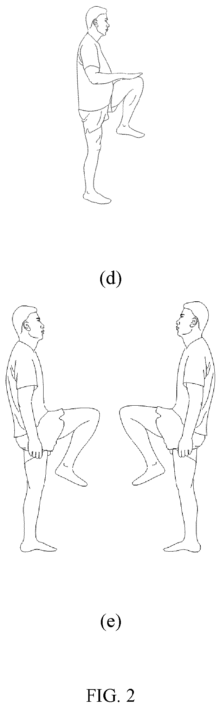

In the embodiment of the present disclosure, the key point detection technology is used to perform key point detection on five sets of video actions of knee and hip joints shown in FIG. 2, and quantize them into 14 detailed action index parameters. It should be noted that, in this embodiment, 14 detailed actions are taken as an example for providing description, but during an implementation, the number of the detailed actions is not limited to 14, and more or less than 14 detailed actions may be adopted.

As shown in FIG. 2, (a) to (e) in FIG. 2 show five sets of video actions of knee and hip joints completed by the user.

Specifically, the (a) in FIG. 2 is a schematic diagram of a video action in which the user completes a crouching (standing-crouching-standing) action. For example, through this video action, an average time duration of the standing-crouching-standing cycle of the user within 20s and an average minimum angle that the knee joint can reach at the time of crouching when the user completes the continuous plurality of actions within 20s can be tested. The shorter the time duration of crouching is, the smaller the knee joint angle at the time of crouching is, it shows that the better the function of the knee j oint is.

The (b) in FIG. 2 is a schematic diagram of a video action in which the user completes a knee joint flexion action in the left and right directions. For example, by completing the video action one time in the left and right directions respectively, a minimum angle that the knee joint can reach when the user completes the right knee flexion action and a minimum angle that the knee joint can reach when the user completes the left knee flexion action may be tested. The smaller the angle is, it shows that the better the function of the knee joint is. In addition, the user may also try this action multiple times to test the changing angle of the calf, and take the best performance as the test result.

The (c) in FIG. 2 is a schematic diagram of a video action in which the user completes the flexion and extension action of the hip joint in the left and right directions. For example, by completing the video action one time in the left and right directions respectively, a maximum angle that the hip joint can reach when the user completes the right hip flexion action, a maximum angle that the hip joint can reach when the user completes the left hip flexion, a maximum angle that the hip joint can reach when the user completes the right hip extension, and a maximum angle that the hip joint can reach when the user completes the left hip extension may be tested. The larger the angle is, it shows that the better the function of the hip joint is.

The (d) in FIG. 2 is a schematic diagram of a video action in which the user completes an action of lifting the knees and alternately patting the knees with the left and right hands. For example, by trying to complete the video action of lifting the knees and alternately patting the knees with the left and right hands for multiple times and with the best performance being taken, a maximum angle which the right leg can reach relative to the vertical direction when lifting the knees and alternately patting the knees with the left and right hands and a maximum angle which the left leg can reach relative to the vertical direction when lifting the knees and alternately patting the knees with the left and right hands may be tested. The greater the angle is, it shows that the better the flexibility function of the hip joint is.

In addition, according to the above video action of lifting the knees and alternately patting the knees with the left and right hands, the number of times of leg lifts when the user lifts his/her left and right legs may be also tested respectively. The more leg lifts there are, it shows that the better the flexibility function of hip joint is.

The (e) in FIG. 2 is a schematic diagram of a video action in which the user completes the action of standing on one foot. For example, by trying multiple times to complete the video action of standing on one foot and lifting the leg on the opposite side and taking a segment with the longest holding action time, a maximum angle which the left leg can reach relative to the vertical direction when the user stands on one foot with the right foot, a maximum angle which the right leg can reach relative to the vertical direction when the user stands on one foot with the left foot, a maintenance time duration when the user stands on one foot with the right foot, and a maintenance time duration when the user stands on one foot with the left foot may be tested. When standing on one foot, the greater the leg lift angle of the opposite leg is, it shows that the better the flexibility function of hip joint is. The longer the time duration of standing on one foot is, it shows that the better the stability function of the lower limbs is.

It should be noted that the above five sets of video actions regarding knee and hip joints and the detailed action index parameters corresponding to the video actions are only examples, and the technology of the present disclosure is not limited to the above video actions and detailed action index parameters, instead any other video actions and corresponding detailed action index parameters that can be used to evaluate the health degree of knee and hip joints may be adopted.

According to the embodiment of the present disclosure, abnormal value determination may be further performed on the plurality of detailed action index parameters, and the detailed action index parameters determined as abnormal values may be removed. For example, the abnormal values in a plurality of detailed action index parameters may be determined based on an IQR (interquartile range) statistical measurement method.

IQR is a commonly used method to detect abnormal values. Specifically, IQR is defined as Q3-Q1, where Q1 is the lower quartile (i.e. the value of 25th percentile) and Q3 is the upper quartile (i.e. the value of 75th percentile). If the value of a data point is less than (Q1-1.5×IQR) or greater than (Q3+1.5×IQR), it is determined as an abnormal value. In addition, if the value of the data point is less than 0, it is replaced by 0, filtering of data noise is completed accordingly.

In the process S 1040, feature importance of a plurality of detailed action index parameters indicating the plurality of detailed actions may be ranked based on a feature screening algorithm, and a certain number of detailed action index parameters may be screened out from the plurality of detailed action index parameters according to a ranking result as a plurality of important index parameters.

According to the embodiment of the present disclosure, the feature screening algorithm may include a Boruta algorithm, and screening out the plurality of important index parameters includes: constructing a plurality of shadow features corresponding to the plurality of detailed action index parameters; ranking feature importance of the plurality of detailed action index parameters with feature importance of the plurality of shadow features as a reference basis; and after multiple iterations, screening out the certain number of detailed action index parameters from the plurality of detailed action index parameters according to the ranking result as the plurality of important index parameters.

In an example, the plurality of detailed action index parameters are taken as original features, the original features are randomized to construct shadow features corresponding to the original features, and then the shadow features and the original features are merged to construct a new extended information data set. The new data set is used to establish a random forest, and importance of each feature (including original features and shadow features) is determined through the random forest. For each original feature, its importance is compared with that of the corresponding shadow feature, wherein the original feature with significantly higher importance than the shadow feature is defined as important, while the original feature with significantly lower importance than the shadow feature is defined as unimportant. Thereafter, the unimportant original features and all shadow features are deleted, and a new round of iteration is carried out again: constructing shadow features - merging into an extended data set - random forest modeling - selecting important features, until all original features are assigned importance, or a preset number of iterations is reached.

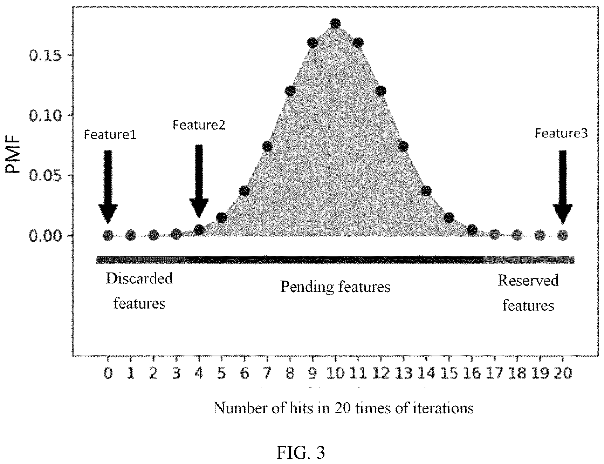

FIG. 3 shows a feature distribution diagram based on a Boruta algorithm according to an embodiment of the present disclosure.

The core of the Boruta algorithm is based on two concepts: Shadow Features and Binomial Distribution. In practice, it is assumed that the original feature is X, each feature in X is randomly shuffle based on the Boruta algorithm, and these randomly shuffled features are called shadow features. Thereafter, the shadow data frame is appended to the original data frame to obtain a new data frame (e.g., X_boruta) whose number of features columns is twice as many as that of the original X. Thereafter, a model is used to train it, and the feature importance of each feature is obtained.

If a feature is useful, its feature importance must be better than that of its random version. In this case, among the original features, it is only necessary to reserve those original features whose feature importance is higher than the highest feature importance score in the random version. However, there may be a problem, this model may not be well trained and the feature importance is not very accurate, so a second strategy, i.e., binomial distribution, is needed.

The core of binomial distribution is continuous iteration. If there is a fluke in one result, it will be iterated for multiple times, such as 20 times and 100 times, and then an average value of feature importance will be taken for comparison and screening. In the Boruta algorithm, the maximum uncertainty level of features is expressed with a 50% probability. Since each independent experiment can give one binary result (hit or miss), a series of n experiments follow binomial distribution, the feature distribution diagram shown in FIG. 3 may be obtained based on the probability mass function of binomial distribution.

In the feature distribution diagram shown in FIG. 3, the left area is a rejection area, and the features of this area are considered as noise, so they may be directly discarded; the middle area is a hesitant area, and it is difficult for the Boruta algorithm to choose the feature of this area; the right area is an acceptable area, and the features of this area are considered to be predictive and may generally be preserved.

In an example, samples of 200 users are collected according to the user collection specification. Based on the Boruta algorithm, the importance of 14 detailed action index parameters of 200 samples is ranked, and the iteration number of the Boruta algorithm is set to 100 times, thus generating a binomial distribution of whether each index parameter meets the standard. Therefore, the index parameters located in the rejection area of data distribution are excluded by a statistical T-Test, and the top eight indexes are screened out as the important index parameters.

In an example, the eight important index parameters may include: an average time duration of standing-crouching-standing, an average minimum angle that the knee joint can reach at the time of crouching, a minimum angle that the knee joint can reach at the time of a right knee bending action, a minimum angle that the knee joint can reach at the time of a left knee bending action, a maximum angle which the right leg can reach relative to the vertical direction when lifting the knees and alternately patting the knees with the left and right hands, a maximum angle which the left leg can reach relative to the vertical direction when lifting the knees and alternately patting the knees with the left and right hands, a maintenance time duration when standing on one foot with the right foot, and a maintenance time duration when standing on one foot with the left foot.

Based on the above eight important index parameters that are screened out, it may be determined that the corresponding video actions regarding knee and hip joints are: crouching, knee joint flexion in the left and right directions, knee lifting and alternately patting the knees with left and right hands, and standing on one foot.

By screening out a plurality of important index parameters from a plurality of detailed action index parameters based on the feature screening algorithm, the actions to be completed by the user when evaluating the health degree of knee and hip joints can be reduced, and the convenience of the user is further improved on the basis of ensuring the accuracy of the evaluation.

In process S1060, the plurality of important index parameters may be inputted into a trained prediction sub-model to output a probability-based prediction result indicating a health degree of joints of each user, wherein a plurality of important index parameters obtained from sample data of part of users among the plurality of users are used as training data to train a prediction sub-model, and a plurality of important index parameters obtained from sample data of remaining users among the plurality of users are used as verification data to test the prediction sub-model to obtain the trained prediction sub-model.

According to the embodiment of the present disclosure, the prediction sub-model may include a LightGBM prediction model, and training of the LightGBM prediction model includes: determining an optimal superparameter combination of the LightGBM prediction model by constructing a plurality of value intervals for a plurality of sets of superparameters of the LightGBM prediction model to run a grid search strategy and by performing k-fold cross-validation on the LightGBM prediction model.

According to the embodiment of the present disclosure, the k-fold cross-validation may include 5-fold cross-validation and 10-fold cross-validation.

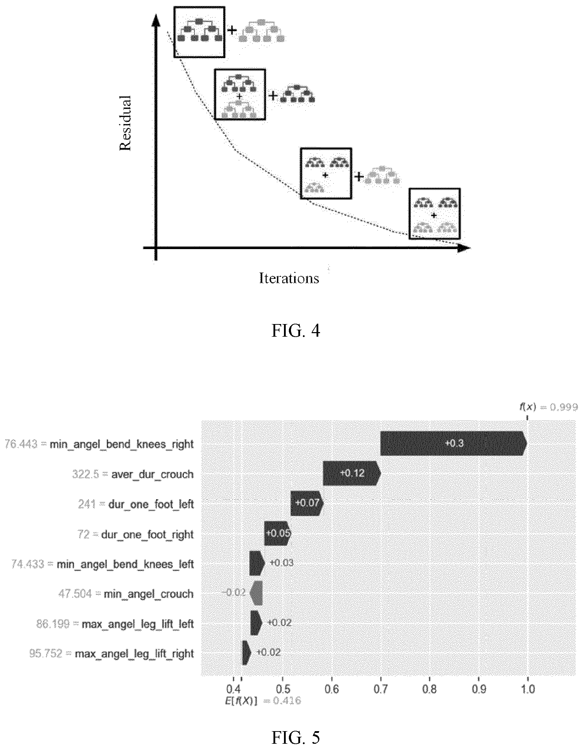

FIG. 4 shows a schematic diagram of a LightGBM prediction model according to an embodiment of the present disclosure.

As shown in FIG. 4, the LightGBM prediction model adopts a decision tree-based integrated machine learning method, and combines multiple weak learners (which usually are decision trees) into a powerful model by using the gradient boosting technology. The principle of the LightGBM prediction model is as follows: Gradient Boosting, the LightGBM prediction model adopts the gradient boosting technology and continuously fits the residual (that is, the difference between the actual value and the predicted value) of the model by way of iteration, thus gradually improving the prediction ability of the model; Decision Tree-Based Model, the LightGBM prediction model uses the decision tree-based model as a weak learner, in which each tree consists of multiple nodes, each node contains one feature and one threshold, by comparing the eigenvalue of the sample with the threshold, the sample is assigned to a left sub-tree or a right sub-tree, so as to realize classification or regression of the sample; Efficient Feature Splitting Strategy, the LightGBM prediction model adopts a feature splitting strategy called "Leaf-wise", which is different from the traditional "Level-wise", it can select the features and thresholds that contribute the most to the current sample set at each splitting, thus reducing the depth of the tree and improving the training speed.

In practice, the original data set is preprocessed and divided into a training set and a testing set for model training and evaluation. In the model training stage, LightGBM is used to train the model on the training set. It is necessary to specify the parameters of the model, such as the learning rate, the number of trees, the depth of trees, the feature splitting strategy, etc. Parameters may be tuned by cross-validation and other methods to obtain better model performance. In the model evaluation stage, the trained model is evaluated on the testing set, and performance of the model may be evaluated by using common evaluation indicators such as accuracy, precision, recall, F1-score, etc. In the model prediction stage, the trained model is used to predict the new data, and the output result of the model may be obtained.

In an example, a LightGBM prediction model is constructed for the eight important index parameters screened out above. For example, for the collected sample data of 200 users, the sample data of 75% users (that is, 150 users) are used as training data to determine the optimal model superparameter structure. Six value intervals are constructed for each of the seven sets of hyperparameters of the model to run the grid search strategy, and the model is verified with 5-fold cross-validation, so as to determine the final optimal combination of hyperparameters of the model. Thereafter, the sample data of the remaining 25% users (that is, 50 users) are used as verification data for testing, and a predicted probability of their sub-health degree is outputted for these 50 users.

By constructing the optimal prediction sub-model based on the plurality of important index parameters that are screened out, it can better evaluate whether the user's knee and hip joints are sub-healthy or healthy, so as to more accurately predict the health degree of the user's knee and hip joints.

In the process S1080, the prediction result may be inputted to an interpretation sub-model to perform a feature contribution degree interpretation on the prediction result, to determine a contribution degree of the plurality of important index parameters to the prediction result.

According to the embodiment of the present disclosure, performing a feature contribution degree interpretation on the prediction result may include: calculating a feature contribution degree of each of the plurality of important index parameters based on a SHAP algorithm; and interpreting a contribution degree of each important index parameter to the prediction result according to the feature contribution degree of each important index parameter.

According to the embodiment of the present disclosure, performing a feature contribution degree interpretation on the prediction result may further include: when the value of the feature contribution degree of an important index parameter is greater than 0, it indicates that the important index parameter has a positive influence on the prediction result; and when the value of the feature contribution degree of the important index parameter is less than 0, it indicates that the important index parameter has a reverse influence on the prediction result, wherein the greater the absolute value of the value of the feature contribution degree of the important index parameter is, the greater the influence of the important index parameter on the prediction result is.

FIG. 5 shows a schematic diagram of a prediction result indicating a health degree of joints of a user and a feature contribution degree of respective important index parameters according to an embodiment of the present disclosure.

Specifically, as can be seen from FIG. 5, the prediction result of the health degree of the user's knee and hip joints is f(x)=0.999, which indicates that the probability of a sub-health degree of the user's knee and hip joints is 99.9%. FIG. 5 further shows the feature contribution degree of the screened out eight important index parameters to the prediction result. The eight important index parameters in FIG. 5 are: an average time duration of standing-crouching-standing (aver_dur_crouch), an average minimum angle that the knee joint can reach at the time of crouching (min angel _crouch), a minimum angle that the knee joint can reach at the time of a right knee bending action (min_angel_bend_knees_right), a minimum angle that the knee joint can reach at the time of a left knee bending action (min_angel_bend_knees_left), a maximum angle which the right leg can reach relative to the vertical direction when lifting the knees and alternately patting the knees with the left and right hands (max_angel_leg_lift_right), a maximum angle which the left leg can reach relative to the vertical direction when lifting the knees and alternately patting the knees with the left and right hands (max_angel_leg_lift_left), a maintenance time duration when standing on one foot with the right foot (dur_one_foot_right), and a maintenance time duration when standing on one foot with the left foot (dur_one_foot_left).

Referring to FIG. 5, taking the index parameter min_angel_bend_knees_right as an example, the index parameter min_angel_bend_knees_right indicates the minimum angle which the right knee can reach at the time of a right knee bending action, and its corresponding feature contribution value is +0.3, which indicates that this index parameter has a positive influence on the prediction result of the sub-health degree of the knee and hip joints, or it may be understood that the index parameter largely causes the sub-health of the user's knee and hip joints. In addition, taking the index parameter min_angel_crouch as an example, the index parameter min_angel_crouch indicates the average minimum angle which the knee joint can reach at the time of crouching, and its corresponding feature contribution value is -0.02, which indicates that this parameter has a negative influence on the prediction result of the sub-health degree of the knee and hip joints, or it may be understood that this index parameter does not lead to the sub-health of the knee and hip joints of the user.

Referring to FIG. 5 again, the index parameters min_angel_bend_knees_right and min_angel_bend_knees_left indicate the minimum angle which the knee joint can reach at the time of a right knee flexion action and the minimum angle which the knee joint can reach at the time of a left knee flexion respectively, their corresponding feature contribution values are +0.3 and +0.03 respectively. That is, the two indexes, min_angel_bend_knees_right and min_angel_bend_knees_left, both have a positive impact on the sub-health of the user's knee and hip joints, or it may be understood as causing the sub-health of the user's knee and hip joints to some extent. In conjunction with (b) in FIG. 2, two index parameters, min_angel_bend_knees_right and min_angel_bend_knees_left, correspond to the video action in which the user completes the knee joint flexion action in the left and right directions as shown in (b) in FIG. 2. Therefore, in conjunction with FIG. 2 and FIG. 5, it may be concluded that the user's knee joint flexion action in the left and right directions reflects the sub-health of the user's knee joint to some extent, the user is reminded that it is necessary to strengthen exercise in this respect.

Through the interpretation sub-model in the present disclosure, further explanation can be made to the user which detailed action indicators and their corresponding actions of knee and hip joints lead to the sub-health of the user's knee and hip joints, thus providing corresponding improvement suggestions and solutions to the user.

In process S 1100, a trained predictive and interpretive model may be obtained based on the trained prediction sub-model and the interpretation sub-model.

According to the predictive and interpretive model of the present disclosure, it can help to adjust the design actions for evaluating a health degree of joints of the user, so as to more accurately evaluate the sub-health degree of joints of the user, and improve the convenience of user operation. In addition, the predictive and interpretive model of the present disclosure includes prediction and interpretation functions, it not only can output the probability-based prediction result for evaluating the health degree of joints of the user, but also can intuitively explain to the user which joints of the body need to be improved.

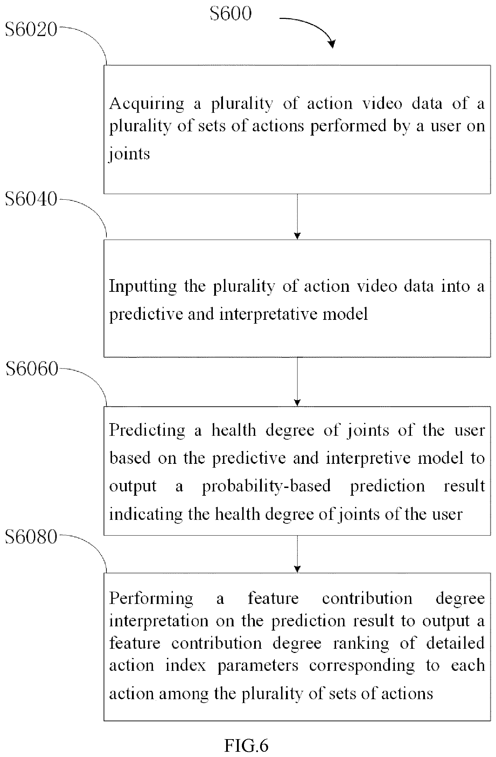

FIG. 6 shows a flowchart S600 of a method for evaluating a health degree of joints according to an embodiment of the present disclosure.

As shown in FIG. 6, the flowchart S600 of a method for evaluating a health degree of joints according to an embodiment of the present disclosure may include respective steps according to processes S6020, S6040, S6060 and S6080.

In the process S6020, a plurality of action video data of a plurality of sets of actions performed by a user on joints may be acquired. For example, the user may complete the corresponding actions according to the five sets of action videos shown in (a) to (e) in FIG. 2.

In the process S6040, the plurality of action video data may be inputted to the predictive and interpretative model obtained according to a training method of a predictive and interpretive model for evaluating a health degree of joints shown in FIG. 1.

In an example, based on the predictive and interpretive model of the embodiment of the present disclosure, 14 detailed action index parameters for the user's knee and hip joints may be calculated through the acquired five sets of action videos, they may include: an average time duration of standing-crouching-standing, an average minimum angle that the knee joint can reach at the time of crouching, a minimum angle that the knee joint can reach at the time of a right knee bending action, a minimum angle that the knee joint can reach at the time of a left knee bending action, a maximum angle that the hip joint can reach at the time of a right hip flexion action, a maximum angle that the hip joint can reach at the time of a left hip flexion action, a maximum angle that the hip joint can reach at the time of a right hip extension action, a maximum angle that the hip joint can reach at the time of a left hip extension action, a maximum angle which the right leg can reach relative to the vertical direction when lifting the knees and alternately patting the knees with the left and right hands, a maximum angle which the left leg can reach relative to the vertical direction when lifting the knees and alternately patting the knees with the left and right hands, a maximum angle which the left leg can reach relative to the vertical direction when standing on one foot with the right foot, a maximum angle which the right leg can reach relative to the vertical direction when standing on one foot with the left foot, a maintenance time duration when standing on one foot with the right foot, and a maintenance time duration when standing on one foot with the left foot.

Thereafter, based on the feature screening function in the predictive and interpretive model of the embodiment of the present disclosure, feature screening is performed on the above 14 detailed action index parameters, and 8 important index parameters are extracted. In an example, the eight important index parameters may include: an average time duration of standing-crouching-standing, an average minimum angle that the knee joint can reach at the time of crouching, a minimum angle that the knee joint can reach at the time of a right knee bending action, a minimum angle that the knee joint can reach at the time of a left knee bending action, a maximum angle which the right leg can reach relative to the vertical direction when lifting the knees and alternately patting the knees with the left and right hands, a maximum angle which the left leg can reach relative to the vertical direction when lifting the knees and alternately patting the knees with the left and right hands, a maintenance time duration when standing on one foot with the right foot, and a maintenance time duration when standing on one foot with the left foot.

In the process S6060, a health degree of joints of the user may be predicted based on the predictive and interpretive model to output a probability-based prediction result indicating the health degree of joints of the user.

In the process S6080, a feature contribution degree interpretation may be performed on the prediction result to output a feature contribution degree ranking of detailed action index parameters corresponding to each action among the plurality of sets of actions.

In conjunction with FIG. 5, with the predictive and interpretive model according to the embodiment of the present disclosure, the prediction result of the sub-health degree of the user's knee and hip joints and the feature contribution of the screened out eight important index parameters to the prediction result can be viewed intuitively.

According to the embodiment of the present disclosure, the influence of respective actions in a plurality of sets of actions on the joint health of the user can be determined based on the prediction result and the feature contribution ranking, and customized sports or exercise suggestions can be further provided for the user.

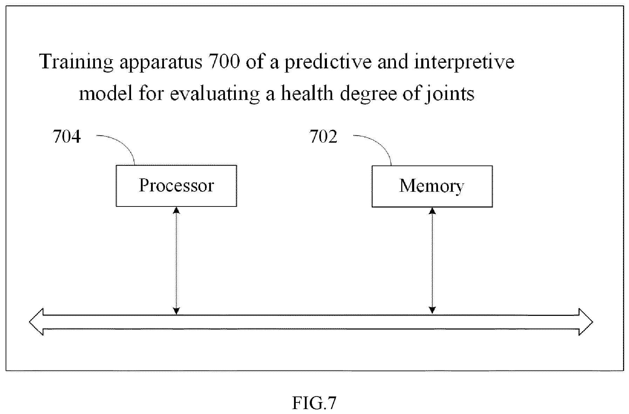

FIG. 7 shows a block diagram of a training apparatus 700 of a predictive and interpretive model for evaluating a health degree of joints according to an embodiment of the present disclosure.

As shown in FIG. 7, the training apparatus 700 for a predictive and interpretive model for evaluating a health degree of joints according to an embodiment of the present disclosure includes: a memory 702 in which computer-executable instructions are stored; and a processor 704 coupled to the memory 702, the processor being configured to: acquire sample data of a plurality of users, wherein sample data of each user includes a plurality of action video data of a plurality of sets of actions performed by each user on joints, and each action of the plurality of sets of actions includes a plurality of detailed actions; rank feature importance of a plurality of detailed action index parameters indicating the plurality of detailed actions based on a feature screening algorithm, and screen out a certain number of detailed action index parameters from the plurality of detailed action index parameters according to a ranking result as a plurality of important index parameters; input the plurality of important index parameters into a trained prediction sub-model to output a probability-based prediction result indicating a health degree of joints of each user, wherein a plurality of important index parameters obtained from sample data of part of users among the plurality of users are used as training data to train a prediction sub-model, and a plurality of important index parameters obtained from sample data of remaining users among the plurality of users are used as verification data to test the prediction sub-model to obtain the trained prediction sub-model; input the prediction result into an interpretation sub-model to perform a feature contribution degree interpretation on the prediction result, to determine a contribution degree of the plurality of important index parameters to the prediction result; and obtain a trained predictive and interpretive model based on the trained prediction sub-model and the interpretation sub-model. The functions performed by the processor 704 in FIG. 7 are the same as or similar to those performed by the processes S1020 to 51100 in FIG. 1, and details will not be repeated here again.

The memory 702 stores computer-executable instructions, which, when executed by the processor 704, realize the training method of the predictive and interpretive model for evaluating the health degree of joints and the computer-implemented method for evaluating the health degree of joints as described above. The memory 702 may be a volatile memory or a nonvolatile memory, or may include both volatile and nonvolatile memories. The nonvolatile memory may be read-only memory (ROM), programmable read-only memory (PROM), erasable programmable read-only memory (EPROM), electrically erasable programmable read-only memory (EEPROM) or flash memory. The volatile memory may be a random access memory (RAM), which is used as an external cache. By way of illustration but not limitation, many forms of RAM are available, such as static random access memory (SRAM), dynamic random access memory (DRAM), synchronous dynamic random access memory (SDRAM), double data rate synchronous dynamic random access memory (DDRSDRAM), enhanced synchronous dynamic random access memory (ESDRAM), synchronous connection dynamic random access memory (SLDRAM) and direct memory bus random access memory (DR RAM). It should be noted that the memories of the methods described herein are intended to include, but are not limited to, these and any other suitable types of memories.

The processor 704 may perform various actions and processes according to programs stored in the memory 702. Specifically, the processor 704 may be an integrated circuit chip with signal processing capability. The processor may be a general processor, a digital signal processor (DSP), an application specific integrated circuit (ASIC), a Field programmable gate array (FPGA) or other programmable logic devices, discrete gate or transistor logic devices, and discrete hardware components. The disclosed methods, steps and logic blocks in the embodiments of the present disclosure may be implemented or performed. The general processor may be a microprocessor or it may be any conventional processor, and it may be X86 architecture or ARM architecture.

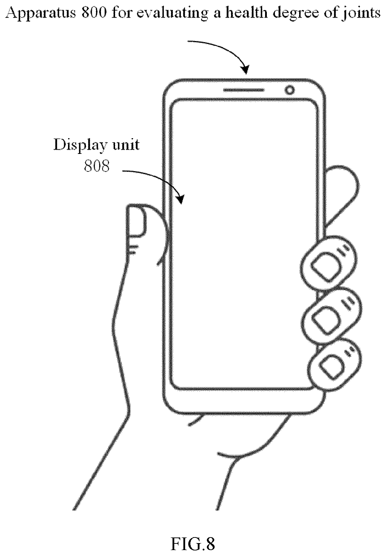

FIG. 8 shows a schematic diagram of an apparatus 800 for evaluating a health degree of joints according to an embodiment of the present disclosure.

As shown in FIG. 8, the apparatus 800 for evaluating a health degree of joints according to an embodiment of the present disclosure includes an acquisition unit 802 (not shown) configured to acquire a plurality of action video data of a plurality of sets of actions performed by a user on joints; an input unit 804 (not shown) configured to input the plurality of action video data into a predictive and interpretative model, wherein the predictive and interpretative model is obtained based on the training method of a predictive and interpretive model for evaluating a health degree of joints shown in FIG. 1; a processing unit 806 (not shown) configured to predict a health degree of joints of the user based on the predictive and interpretive model to output a probability-based prediction result indicating the health degree of joints of the user, the processing unit 806 is further configured to perform a feature contribution degree interpretation on the prediction result to output a feature contribution degree ranking of detailed action index parameters corresponding to each action among the plurality of sets of actions; and a display unit 808 configured to display the prediction result and the feature contribution ranking.

The above acquisition unit involved in the present disclosure may acquire a plurality of action video data of a plurality of sets of actions that a user is performing on joints in real time. For example, the acquisition unit of the apparatus may be a camera, and a plurality of action video data for a plurality of actions are acquired by shooting videos of a plurality of actions being performed by a user on joints in real time. Alternatively, the user may locally upload the videos of the previously completed plurality of sets of actions on joints to the apparatus for evaluating a health degree of joints according to the embodiment of the present disclosure, and then a plurality of action video data are acquired from the plurality of sets of actions through the acquisition unit in the apparatus. In addition, the apparatus for evaluating a health degree of joints according to the embodiment of the present disclosure may communicate with a device such as a server to acquire videos of the plurality of sets of actions performed by a user on joints from the server through the acquisition unit of the apparatus.

The apparatus for evaluating a health degree of joints according to the present disclosure may be any type of portable device (such as a smart phone, a tablet computer, etc.) or any type of fixed device (such as a desktop computer, etc.). Taking the smart phone as an example, the user can intuitively view whether the joints are in a sub-healthy state and learn which aspects of exercise should be focused on by outputting the prediction result indicating the health degree of joints of the user and the influence of respective actions in the plurality of sets of actions performed by the user on the health degree of joints of the user to a display interface of the user's mobile phone.

In addition, the above training method of a predictive and interpretive model for evaluating a health degree of joints of the present disclosure and the above computer-implemented method for evaluating a health degree of joints may be stored in a computer program product. Specifically, according to the present disclosure, it is possible to provide a computer program product storing computer-readable instructions, which, when executed by a processor, can cause a processor to implement the training method of a predictive and interpretive model for evaluating a health degree of joints of the present disclosure and the computer-implemented method for evaluating a health degree of joints as described above.

It should be noted that, the flowcharts and block diagrams in the drawings illustrate the architecture, functionality, and operation of possible implementations of systems, methods and computer program products according to various embodiments of the present invention. In this regard, each block in the flowcharts or block diagrams may represent a module, a program segment, or a portion of code, the module, the program segment, or the portion of code includes at least one executable instruction for implementing the specified logical function. It should also be noted that, in some alternative implementations, the functions noted in the block may occur out of the order noted in the drawings. For example, two blocks shown in succession may, in fact, be executed substantially concurrently, or they may sometimes be executed in the reverse order, depending upon the functionality involved. It will also be noted that, each block of the block diagrams and/or flowcharts, and combinations of blocks in the block diagrams and/or flowcharts, may be implemented by special-purpose hardware-based systems that perform the specified functions or operations, or by combinations of special-purpose hardware and computer instructions.

Generally, various example embodiments of the present disclosure may be implemented in hardware or dedicated circuits, software, firmware, logic, or any combinations thereof. Some aspects may be implemented in hardware, while other aspects may be implemented in firmware or software that can be executed by a controller, a microprocessor or other computing devices. While aspects of the embodiments of the present disclosure are illustrated or described as block diagrams, flow charts, or some other image representations, it will be understood that the blocks, devices, systems, techniques, or methods described herein may be implemented in hardware, software, firmware, special-purpose circuits or logic, general-purpose hardware or controllers or other computing devices, or some combinations thereof, as non-limiting examples.

Unless otherwise defined, all terms (including technical and scientific terms) used herein have the same meaning as commonly understood by those of ordinary skill in the art to which this disclosure belongs. It will be further understood that terms, such as those defined in commonly used dictionaries, should be interpreted as having the meaning that is consistent with their meaning in the context of the relevant art and will not be interpreted in an idealized or overly formal sense unless expressly so defined herein.

The above is illustration of the present disclosure and should not be construed as making limitation thereto. Although some exemplary embodiments of the present disclosure have been described, those skilled in the art can easily understand that many modifications may be made to these exemplary embodiments without departing from the creative teaching and advantages of the present disclosure. Therefore, all such modifications are intended to be included within the scope of the present disclosure as defined by the appended claims. As will be appreciated, the above is to explain the present disclosure, it should not be constructed as limited to the specific embodiments disclosed, and modifications to the embodiments of the present disclosure and other embodiments are intended to be included in the scope of the attached claims. The present disclosure is defined by the claims and their equivalents.

## Claims

1. A training method of a predictive and interpretive model for evaluating a health degree of joints, the method comprising:
acquiring sample data of a plurality of users, wherein sample data of each user comprises a plurality of action video data of a plurality of sets of actions performed by each user on joints, and each action of the plurality of sets of actions comprises a plurality of detailed actions;
ranking feature importance of a plurality of detailed action index parameters indicating the plurality of detailed actions based on a feature screening algorithm, and screening out a certain number of detailed action index parameters from the plurality of detailed action index parameters according to a ranking result as a plurality of important index parameters;
inputting the plurality of important index parameters into a trained prediction sub-model to output a probability-based prediction result indicating a health degree of joints of each user, wherein a plurality of important index parameters obtained from sample data of part of users among the plurality of users are used as training data to train a prediction sub-model, and a plurality of important index parameters obtained from sample data of remaining users among the plurality of users are used as verification data to test the prediction sub-model to obtain the trained prediction sub-model;
inputting the prediction result into an interpretation sub-model to perform a feature contribution degree interpretation on the prediction result, to determine a contribution degree of the plurality of important index parameters to the prediction result; and
obtaining a trained predictive and interpretive model based on the trained prediction sub-model and the interpretation sub-model.

2. The method according to claim 1, further comprising: performing action detection on each of the plurality of action video data through a key point detection algorithm to obtain the quantized plurality of detailed action index parameters indicating different detailed actions in the plurality of sets of actions.

3. The method according to claim 1, further comprising: performing abnormal value determination on the plurality of detailed action index parameters, and removing the detailed action index parameters determined as abnormal values.

4. The method according to claim 3, wherein abnormal value determination is performed on the plurality of detailed action index parameters based on an IQR statistical measurement method, and the IQR statistical measurement method comprises:
calculating a first value by subtracting 1.5 IQR from the 25th percentile value and a second value by adding 1.5 IQR to the 75th percentile value, and determining a value smaller than the first value and a value larger than the second value as the abnormal values, wherein IQR refers to a value obtained by subtracting the 25th percentile value from the 75th percentile value.

5. The method according to claim 1, wherein the feature screening algorithm comprises a Boruta algorithm, and screening out the plurality of important index parameters comprises:
constructing a plurality of shadow features corresponding to the plurality of detailed action index parameters;
ranking feature importance of the plurality of detailed action index parameters with feature importance of the plurality of shadow features as a reference basis; and
after multiple iterations, screening out the certain number of detailed action index parameters from the plurality of detailed action index parameters according to the ranking result as the plurality of important index parameters.

6. The method according to claim 1, wherein the prediction sub-model comprises a LightGBM prediction model, and training of the LightGBM prediction model comprises:
determining an optimal superparameter combination of the LightGBM prediction model by constructing a plurality of value intervals for a plurality of sets of superparameters of the LightGBM prediction model to run a grid search strategy and by performing k-fold cross-validation on the LightGBM prediction model.

7. The method according to claim 6, wherein the k-fold cross-validation comprises 5-fold cross-validation and 10-fold cross-validation.

8. The method according to claim 1, wherein performing a feature contribution degree interpretation on the prediction result comprises:
calculating a feature contribution degree of each of the plurality of important index parameters based on a SHAP algorithm; and
interpreting a contribution degree of each important index parameter to the prediction result according to the feature contribution degree of each important index parameter.

9. The method according to claim 8, wherein performing a feature contribution degree interpretation on the prediction result further comprises:
when the value of the feature contribution degree of an important index parameter is greater than 0, it indicates that the important index parameter has a positive influence on the prediction result; and
when the value of the feature contribution degree of the important index parameter is less than 0, it indicates that the important index parameter has a reverse influence on the prediction result,
wherein the greater the absolute value of the value of the feature contribution degree of the important index parameter, the greater the influence of the important index parameter on the prediction result.

10. The method according to claim 1, wherein the joints comprise at least one or more of shoulder j oint, elbow joint, hip joint, wrist j oint, knee joint and ankle joint.

11. The method according to any one of claims 1 to 10, wherein the plurality of sets of actions at least comprise: crouching, knee joint flexion in the left and right directions, hip joint flexion and extension in the left and right directions, knee lifting and alternately patting the knees with left and right hands, and standing on one foot.

12. The method according to claim 11, wherein the plurality of detailed action index parameters at least comprise: an average time duration of standing-crouching-standing, an average minimum angle that the knee joint can reach at the time of crouching, a minimum angle that the knee joint can reach at the time of a right knee bending action, a minimum angle that the knee joint can reach at the time of a left knee bending action, a maximum angle that the hip joint can reach at the time of a right hip flexion action, a maximum angle that the hip joint can reach at the time of a left hip flexion action, a maximum angle that the hip joint can reach at the time of a right hip extension action, a maximum angle that the hip joint can reach at the time of a left hip extension action, a maximum angle which the right leg can reach relative to the vertical direction when lifting the knees and alternately patting the knees with the left and right hands, a maximum angle which the left leg can reach relative to the vertical direction when lifting the knees and alternately patting the knees with the left and right hands, a maximum angle which the left leg can reach relative to the vertical direction when standing on one foot with the right foot, a maximum angle which the right leg can reach relative to the vertical direction when standing on one foot with the left foot, a maintenance time duration when standing on one foot with the right foot, and a maintenance time duration when standing on one foot with the left foot.

13. The method according to claim 12, wherein the plurality of important index parameters at least comprise: an average time duration of standing-crouching-standing, an average minimum angle that the knee joint can reach at the time of crouching, a minimum angle that the knee joint can reach at the time of a right knee bending action, a minimum angle that the knee joint can reach at the time of a left knee bending action, a maximum angle which the right leg can reach relative to the vertical direction when lifting the knees and alternately patting the knees with the left and right hands, a maximum angle which the left leg can reach relative to the vertical direction when lifting the knees and alternately patting the knees with the left and right hands, a maintenance time duration when standing on one foot with the right foot, and a maintenance time duration when standing on one foot with the left foot.

14. A computer-implemented method for evaluating a health degree of joints, the method comprising:
acquiring a plurality of action video data of a plurality of sets of actions performed by a user on joints;
inputting the plurality of action video data into a predictive and interpretative model;
predicting a health degree of joints of the user based on the predictive and interpretive model to output a probability-based prediction result indicating the health degree of joints of the user; and
performing a feature contribution degree interpretation on the prediction result to output a feature contribution degree ranking of detailed action index parameters corresponding to each action among the plurality of sets of actions,
wherein the predictive and interpretive model is obtained based on the method according to any one of claims 1 to 13.

15. The method of claim 14, further comprising:
determining an influence of respective actions among the plurality of sets of actions on the health degree of joints of the user, based on the prediction result and the feature contribution ranking.

16. The method according to claim 14 or 15, further comprising:
providing customized sports or exercise suggestions for the user according to the prediction result and the feature contribution ranking.

17. A training apparatus for a predictive and interpretive model for evaluating a health degree of joints, the apparatus comprising:
a memory in which computer-executable instructions are stored; and
a processor coupled to the memory, the processor being configured to:
acquire sample data of a plurality of users, wherein sample data of each user comprises a plurality of action video data of a plurality of sets of actions performed by each user on joints, and each action of the plurality of sets of actions comprises a plurality of detailed actions;
rank feature importance of a plurality of detailed action index parameters indicating the plurality of detailed actions based on a feature screening algorithm, and screen out a certain number of detailed action index parameters from the plurality of detailed action index parameters according to a ranking result as a plurality of important index parameters;
input the plurality of important index parameters into a trained prediction sub-model to output a probability-based prediction result indicating a health degree of joints of each user, wherein a plurality of important index parameters obtained from sample data of part of users among the plurality of users are used as training data to train a prediction sub-model, and a plurality of important index parameters obtained from sample data of remaining users among the plurality of users are used as verification data to test the prediction sub-model to obtain the trained prediction sub-model;
input the prediction result into an interpretation sub-model to perform a feature contribution degree interpretation on the prediction result, to determine a contribution degree of the plurality of important index parameters to the prediction result; and
obtain a trained predictive and interpretive model based on the trained prediction sub-model and the interpretation sub-model.

18. An apparatus for evaluating a health degree of joints, the apparatus comprising:
an acquisition unit configured to acquire a plurality of action video data of a plurality of sets of actions performed by a user on joints;
an input unit configured to input the plurality of action video data into a predictive and interpretative model, wherein the predictive and interpretative model is obtained based on the method of any one of claims 1 to 13;
a processing unit configured to predict a health degree of joints of the user based on the predictive and interpretive model to output a probability-based prediction result indicating the health degree of joints of the user,
the processing unit is further configured to perform a feature contribution degree interpretation on the prediction result to output a feature contribution degree ranking of detailed action index parameters corresponding to each action among the plurality of sets of actions; and
a display unit configured to display the prediction result and the feature contribution ranking.

19. A computer program product, wherein computer-readable instructions, which implement the method according to any one of claims 1 to 16 when executed by a processor, are stored on the computer program product.
